# EUROPEAN PATENT APPLICATION

(11) **EP 4 455 275 A1**
(43) Date of publication of application: **30.10.2024**
(21) Application number: 22911375.8
(22) Date of filing: 22.12.2022
(51) Int. Cl.: C12N 5/0786, C12M 3/00, C12N 5/0784, C12N 5/0789

(54) **METHOD FOR PRODUCING MACROPHAGES, DIFFERENTIATION INDUCING AGENT, DIFFERENTIATION INDUCTION KIT, METHOD FOR CULTURING MACROPHAGES, AGENT FOR PROMOTING MACROPHAGE PROPAGATION, KIT FOR PROMOTING MACROPHAGE PROLIFERATION, METHOD FOR MACROPHAGE PROLIFERATION, AND MACROPHAGES**

(30) Priority: 22.12.2021 JP 2021208420
(71) Applicant: Kagoshima University, Kagoshima-shi, Kagoshima 890-8580 (JP)
(72) Inventor: HARA, Hiromitsu, Kagoshima-shi, Kagoshima 890-8580 (JP); MATSUMOTO, Shin-ei, Kagoshima-shi, Kagoshima 890-8580 (JP); TOYONAGA, Kenji, Kagoshima-shi, Kagoshima 890-8580 (JP)
(74) Representative: v. Bezold & Partner Patentanwälte - PartG mbB
(86) International application number: PCT/JP2022/047472
(87) International publication number: WO 2023/120673

(57) **Abstract**

A method of producing macrophages includes a culture step of culturing hematopoietic progenitor cells in the presence of a TREM2 signal activator.

## Description

### Technical Field

The present disclosure relates to a method of producing macrophages, an agent for differentiation induction, a kit for differentiation induction, a method of culturing macrophages, an agent for promoting proliferation of macrophages, a kit for promoting proliferation of macrophages, a method of proliferating macrophages, and a macrophage.

### Background Art

Macrophages are leukocytes that contribute to protection against infections, and maintenance of tissue homeostasis. Macrophages in a living body are derived from hematopoietic stem cells in the bone marrow, and their differentiation requires stimulation by macrophage colony-stimulating factor (M-CSF) or granulocyte-macrophage colony-stimulating factor (GM-CSF). In fact, a method of inducing differentiation of cells having characteristics of monocytes and macrophages (bone marrow derived macrophages: BMDMs) by culturing mouse bone marrow cells in vitro in the presence of M-CSF has been established. BMDMs are widely used as a tool to study macrophages in vitro.

BMDMs have, for example, the following problems: i) since M-CSF, which is used for the induction, is expensive, the cost of large-scale culture of BMDMs is high; ii) BMDMs can survive for only about one week; iii) the number of cells that can be obtained by a single time of culture is limited since their proliferation stops after the differentiation; and iv) due to the problem of iii), the cells cannot be re-cultured in cases where they are thawed after cryopreservation. Besides BMDMs, various macrophage cell lines derived from rodents and humans are frequently used in in vitro studies. Although these cells do not have the problems of i) to iv) described above, they are tumor cells, and hence their use in in vivo tests such as administration to a living body is restricted.

As an agent for differentiation induction of macrophages from monocytes without use of M-CSF, Patent Literature 1 discloses an agent for differentiation induction, the agent including a compound having antagonistic action on angiotensin II receptor, and activating action on peroxisomal proliferator-activated receptor γ.

### Citation List

### Patent Literature

Patent Literature 1: Unexamined Japanese Patent Application Publication No. 2015-47125

### Non Patent Literature

Non Patent Literature 1: Hadas Keren-Shaul and 13 others, "A Unique Microglia Type Associated with Restricting Development of Alzheimer's Disease", Cell, 2017, 169, 1276-1290
Non Patent Literature 2: Diego Adhemar Jaitin and 22 others, "Lipid-Associated Macrophages Control Metabolic Homeostasis in a Trem2-Dependent Manner", Cell, 2019, 178, 686-698

### Summary of Invention

### Technical Problem

However, in Patent Literature 1, there is neither a study on the survival period of macrophages obtained by the agent for differentiation induction, nor a study on the proliferation properties and whether or not cryopreservation is possible after the differentiation.

The present disclosure was carried out in view of the above circumstances, and an objective of the present disclosure is to provide a method of producing macrophages, an agent for differentiation induction, a kit for differentiation induction, a method of culturing macrophages, an agent for promoting proliferation of macrophages, a kit for promoting proliferation of macrophages, and a method of proliferating macrophages that enable production of macrophages having a long survival period and a long proliferation period, the macrophages being cryopreservable and applicable to a wide range of uses as a research tool. Another obj ective of the present disclosure is to provide a macrophage having a long survival period and a long proliferation period, the macrophage being cryopreservable and applicable to a wide range of uses as a research tool.

### Solution to Problem

The onset of a disease causes remodeling of immune cells, and this results in appearance of macrophage populations having special characteristics in a tissue. Such macrophage populations are known to play important roles in exacerbation or suppression of the disease. For example, activated microglial cells (disease-associated microglia: DAM), which exhibit special characteristics, are known to appear in the brain of a mouse model of Alzheimer's disease (see Non Patent Literature 1). In adipose tissues of mice that have developed lifestyle-related diseases due to a high-fat diet, macrophage populations that are absent in a normal state (lipid-associated macrophages: LAMs) appear (see Non Patent Literature 2). DAMs and LAMs are among the molecules that are characteristically highly expressed in the lipid-recognizing receptor TREM2 (triggering receptor expressed on myeloid cells 2). Non Patent Literatures 1 and 2 show that TREM2 is deeply involved in the induction of these disease-associated macrophages.

The present inventors discovered that stimulation of hematopoietic progenitor cells with a TREM2 signal activator causes appearance of macrophage-like cells, thereby completing the present disclosure.

### (Method of Producing Macrophages)

The method of producing macrophages, described herein includes a culture step of culturing hematopoietic progenitor cells in the presence of a TREM2 signal activator.

The TREM2 signal activator may be a lipid.

The TREM2 signal activator may be a lipid derived from brain or a lipid containing two long-chain fatty groups.

The TREM2 signal activator may be a compound represented by Formula (I): 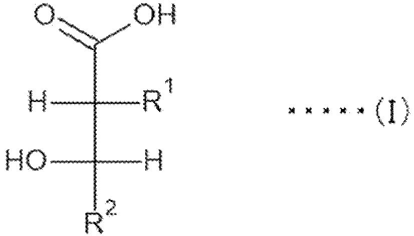
(wherein the compound has 60 to 90 carbon atoms; R¹ represents a saturated or unsaturated aliphatic hydrocarbon group; and R² represents a saturated or unsaturated aliphatic hydrocarbon group optionally containing a ring structure or a substituent),
an ester thereof, or a salt thereof.

A cell culture vessel for culturing the hematopoietic progenitor cells may have a surface coated with the TREM2 signal activator.

The hematopoietic progenitor cells may be myeloid progenitor cells.

The hematopoietic progenitor cells may be macrophage and dendritic cell progenitors, or common monocyte progenitors.

### (Agent for Differentiation Induction into Macrophages)

The agent for differentiation induction from hematopoietic progenitor cells into macrophages, described herein includes a TREM2 signal activator.

### (Kit for Differentiation Induction into Macrophages)

The kit for differentiation induction from hematopoietic progenitor cells into macrophages, described herein includes a cell culture vessel having a surface coated with a TREM2 signal activator.

### (Method of Culturing Macrophages)

The method of culturing macrophages, described herein includes a culture step of culturing hematopoietic progenitor cells in the presence of a TREM2 signal activator.

### (Agent for Promoting Proliferation of Macrophages)

The agent for promoting proliferation of macrophages, described herein includes a TREM2 signal activator.

### (Kit for Promoting Proliferation of Macrophages)

The kit for promoting proliferation of macrophages, described herein includes a cell culture vessel having a surface coated with a TREM2 signal activator.

### (Method of Proliferating Macrophages)

The method of proliferating macrophages, described herein includes a culture step of culturing macrophages in presence of a TREM2 signal activator.

### (Macrophages)

The macrophage described herein has proliferative capacity after the 10th of subculture in a medium containing neither a macrophage colony-stimulating factor nor a granulocyte-macrophage colony-stimulating factor, but containing a TREM2 signal activator.

The macrophage described herein is a macrophage whose differentiation is to be induced dependently on TREM2 by culturing hematopoietic progenitor cells in a medium containing neither a macrophage colony-stimulating factor nor a granulocyte-macrophage-stimulating factor, but containing a TREM2 signal activator.

The macrophage described herein has, after cryopreservation, proliferative capacity in a medium containing neither a macrophage colony-stimulating factor nor a granulocyte-macrophage colony-stimulating factor, but containing a TREM2 signal activator.

The macrophage described herein is a macrophage whose culture supernatant after 24 hours of culture in a medium supplemented with lipopolysaccharide contains TNF-α, IL-6, and nitric oxide at concentrations of not more than 30% as compared to bone marrow derived macrophages induced from bone marrow cells with macrophage colony-stimulating factor.

The macrophage described herein is a macrophage wherein expression of one or more genes selected from the genes listed in Table 1 below is higher than in at least one selected from an alveolar macrophage, a BMDM, a BMDM induced to M1, a BMDM induced to M2, a Kupffer cell, a microglial cell, an osteoclast, and a peritoneal exudate macrophage.

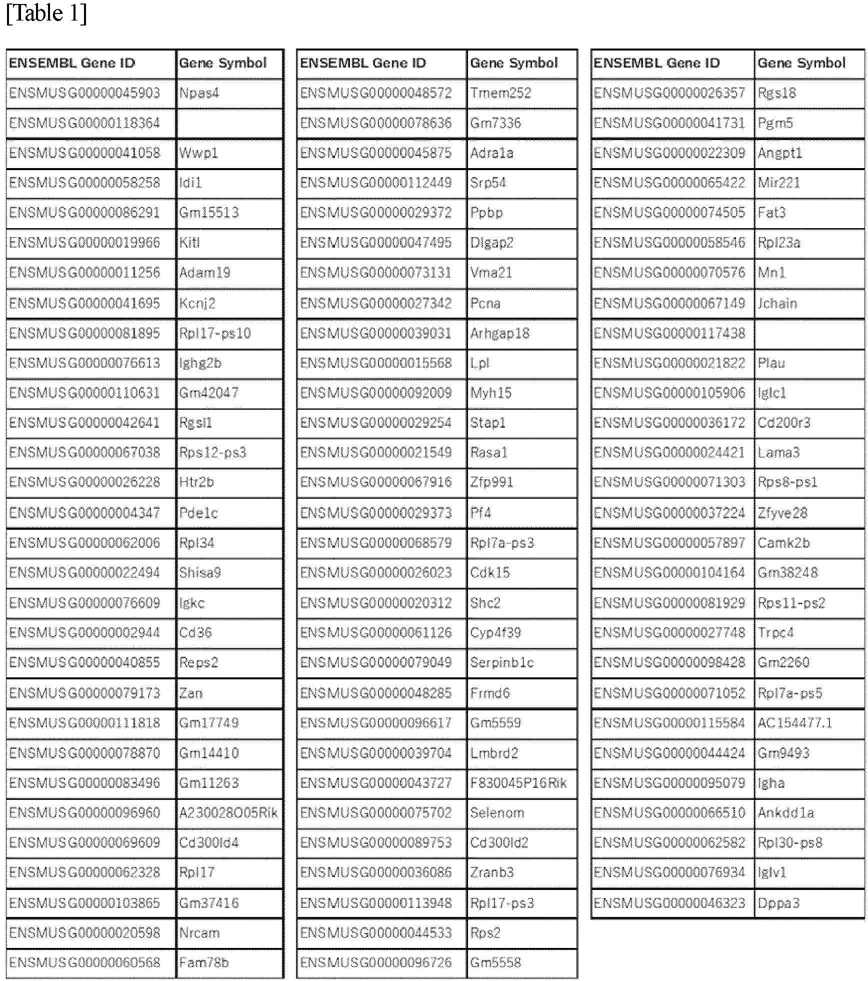

The macrophage described herein is a macrophage wherein expression of one or more genes selected from the genes listed in Table 2 below is lower than in at least one selected from an alveolar macrophage, a BMDM, a BMDM induced to M1, a BMDM induced to M2, a Kupffer cell, a microglial cell, an osteoclast, and a peritoneal exudate macrophage.

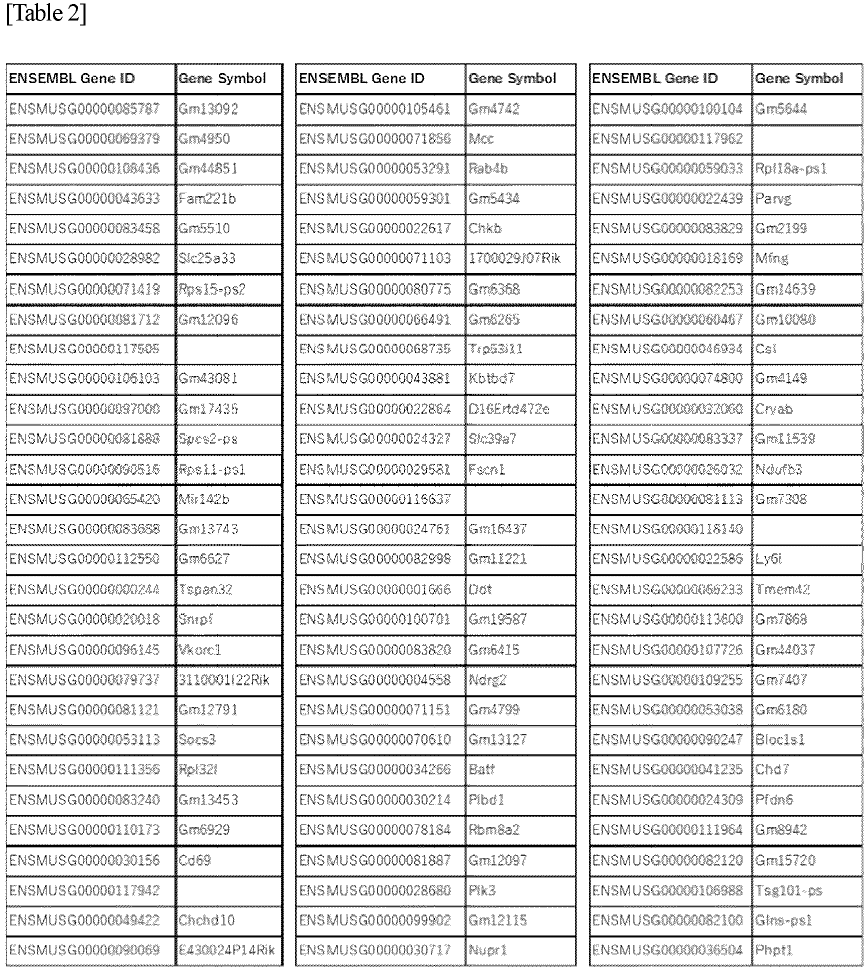

### Advantageous Effects of Invention

By the present disclosure, a macrophage having a long survival period and a long proliferation period, the macrophage being cryopreservable and applicable to a wide range of uses as a research tool, can be obtained.

### Brief Description of Drawings

FIG. 1 is a diagram illustrating images of macrophages obtained by differentiation induction from mouse bone marrow cells; Panel (A) is an image of macrophages obtained by differentiation induction with brain lipid; Panel (B) is an image of macrophages obtained by differentiation induction with M-CSF;
FIG. 2 is a diagram illustrating dot plots obtained by the flow cytometric analysis according to Test Example 2;
FIG. 3 is a diagram illustrating univariate histograms of surface antigens obtained by the flow cytometric analysis according to Test Example 2;
FIG. 4 is a diagram illustrating the number of cells with respect to the mass of brain lipid per unit culture area, according to Test Example 3;

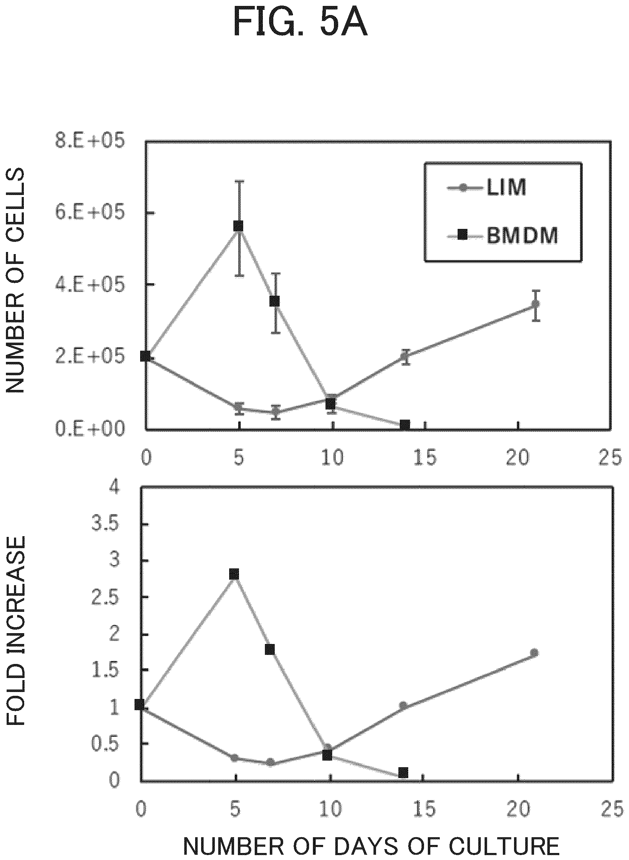

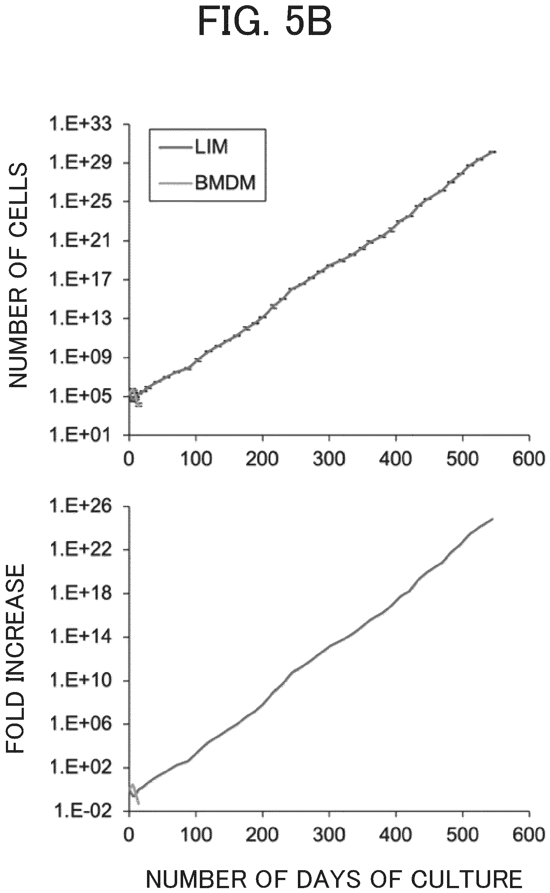

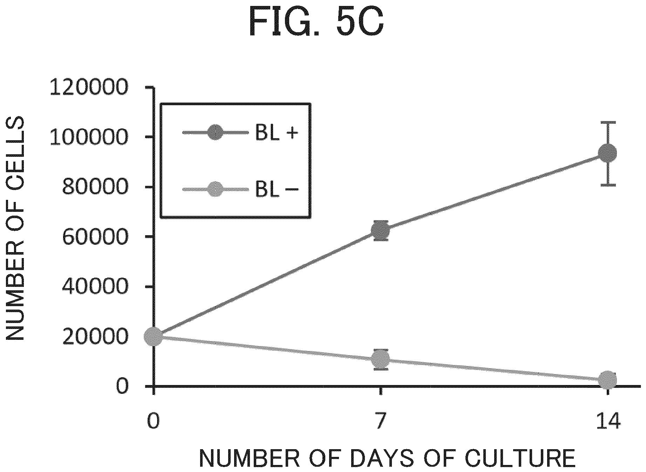

FIG. 5 is a diagram illustrating changes in the number of cells over time, according to Test Example 4; the top row of Panel (A) illustrates the number of cells on Days 0 to 21 of culture; the bottom row of Panel (A) illustrates the fold increases in the number of cells during Days 0 to 21 of culture relative to the number of cells on Day 0, which was taken as 1; the top row of Panel (B) illustrates the number of cells on Days 0 to 544 of culture; the bottom row of Panel (B) illustrates the fold increases in the number of cells during Days 0 to 544 of culture relative to the number of cells on Day 0, which was taken as 1; Panel (C) illustrates the number of macrophage cells cultured in the presence or absence of brain lipid;
FIG. 6 is a diagram illustrating changes in the number of macrophage cells over time according to Test Example 5, wherein the macrophages were thawed after cryopreservation;
FIG. 7 is a diagram illustrating the number of macrophages after stimulation with brain lipid according to Test Example 6; Panel (A) illustrates the number of macrophages that have differentiated from Lin-positive cells and Lin-negative cells in mixed culture; Panel (B) illustrates the number of macrophages that have differentiated from Lin-positive cells or Lin-negative cells that were cultured alone;
FIG. 8 is a diagram illustrating the number of viable cells after induction of differentiation of each type of mouse bone marrow cells, according to Test Example 7;
FIG. 9 is a diagram illustrating the ligand activity as observed using TREM2 reporter cells, according to Test Example 8;
FIG. 10 is a diagram illustrating the number of viable cells after induction of differentiation of mouse bone marrow cells with each test substance, according to Test Example 8;
FIG. 11 is a diagram illustrating the phagocytotic ability of macrophages, according to Test Example 9; and
FIG. 12 is a diagram illustrating the cytokine production response of macrophages to lipopolysaccharide (LPS) stimulation, according to Test Example 10; Panels (A), (B), (C), (D), and (E) illustrate the concentrations of monocyte chemoattractant protein-1 (MCP-1), TNF-α, IL-6, IL-10, and nitric oxide (NO), respectively.

### Description of Embodiments

Embodiments according to the present disclosure are described with reference to drawings. The present disclosure is not limited by the following embodiments.

The method of producing macrophages according to the present embodiment includes a culture step of culturing hematopoietic progenitor cells in the presence of a TREM2 signal activator. TREM2 is an immunoglobulin superfamily receptor that associates with the adaptor molecule DAP12 (DNAX-activating protein of 12 kDa). The TREM2 signal activator may be any substance as long as it specifically binds to TREM2 to generate a signal via DAP12.

The TREM2 signal activator is, for example, an antibody or an antigen-binding fragment thereof, a compound, or a ligand of TREM2, having TREM2 agonist activity or DAP12 agonist activity. The antibody may be either a polyclonal antibody or a monoclonal antibody. Further, the antibody may be a human-type chimeric antibody, a humanized antibody, or a human antibody. The antibody herein includes antigen binding of an antibody. The antigen-binding fragment is a protein or a peptide containing a portion (partial fragment) of an antibody, and retaining the ability of the antibody to act on and bind to an antigen. Examples of the antigen-binding fragment include F(ab')2, Fab', Fab, Fab3, single-chain Fv (scFv), (tandem) bispecific single-chain Fv (sc(Fv)2), single-chain triplebody, nanobody, divalent VHH, pentavalent VHH, minibody, (double-chain) diabody, tandem diabody, bispecific tribody, bispecific bibody, dual affinity retargeting molecule (DART), triabody (or tribody), tetrabody (or [sc(Fv)2]2), and disulfide-bonded Fv (dsFv), and their polymers (see Nature Biotechnology, 29(1): 5-6 (2011); Maneesh Jain and others, TRENDS in Biotechnology, 25(7) (2007): 307-316; and Christoph Stein and others, Antibodies (1): 88-123 (2012)).

The TREM2 signal activator is, for example, a ligand of TREM2 or a lipid. A lipid is an organic compound that is insoluble in water and readily soluble in organic solvents. The lipid is not limited, and may be either a liquid or a solid at normal temperature. Examples of the lipid include sphingoglycolipids, phospholipids, sulfated glycolipids, and fatty acids. The lipid is preferably a lipid having two long-chain fatty groups. Examples of the long-chain fatty acid groups include fatty acid groups having 6 to 60, or 10 to 50 carbon atoms. The number of the carbon atoms constituting the carbon chains of the long-chain fatty acid groups may be either the same or different.

The fatty acid may be either a saturated fatty acid or an unsaturated fatty acid. Preferred examples of the lipid include 2-tetradecylhexadecanoic acid (THA), β-glucosylceramide (β-GluCer), α-galactosylceramide (α-GalCer), phosphatidylcholine (PC), lysophosphatidylcholine (LPC), phosphatidylethanolamine (PE), sulfatide (Sulf), and mycolic acid (MA). The TREM2 signal activator may be a mixture of a plurality of kinds of lipids, such as the whole lipid extracted from an animal-derived tissue such as brain, from a plantderived tissue, or from a microorganism including bacteria using an organic solvent, or a synthetic lipid that binds to TREM2. One aspect of the TREM2 signal activator is a cell or a microorganism containing a lipid on the cell surface. The TREM2 signal activator is preferably a lipid derived from brain, or MA.

The TREM2 signal activator may be a compound represented by Formula (I), an ester thereof, or a salt thereof. The number of carbon atoms in one molecule of the compound is, for example, 60 to 90, or 70 to 80. R¹ represents a saturated or unsaturated aliphatic hydrocarbon group. The number of carbon atoms of R¹ is, for example, 6 to 60, 10 to 50, 20 to 40, 22 to 30, or 22 to 26. R² represents a saturated or unsaturated aliphatic hydrocarbon group that may contain a ring structure or a substituent. The number of carbon atoms of R² is, for example, 6 to 60, 10 to 50, 20 to 40, 22 to 30, or 22 to 26. The number of carbon atoms constituting the ring structure is, for example, 3 to 6. The ring structure may be contained as a cycloalkylene group in R², or as a cycloalkyl group in R². R² may contain one to several ring structures. Examples of the substituent include, but are not limited to, methyl, hydroxy, carbonyl, and carboxyl.

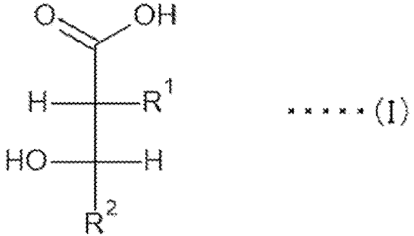

The salt of the compound represented by Formula (I) is not limited as long as it is a pharmaceutically acceptable salt, and may be either an acidic salt or a basic salt. Examples of the salt include alkali metal salts such as lithium salt, sodium salt, and potassium salt; alkaline earth metal salts such as magnesium salt and calcium salt; inorganic acid salts such as hydrochloride, hydrobromide, hydroiodide, sulfate, nitrate, and phosphate; and organic acid salts such as formate, acetate, oxalate, propionate, hexanoate, cyclopentanepropionate, glycolate, pyruvate, lactate, malonate, succinate, malate, fumarate, tartrate, dibenzoyltartrate, ditoluoyltartrate, citrate, benzoate, o-(4-hydroxybenzoyl)benzoate, cinnamate, mandelate, methanesulfonate, ethanesulfonate, 1,2-ethanedisulfonate, 2-hydroxyethanesulfonate, benzenesulfonate, p-chlorobenzenesulfonate, 2-naphthalenesulfonate, p-toluenesulfonate, aspartate, camphorsulfonate, glucoheptanoate, 3-phenylpropionate, trimethylacetate, *tert*-butyl acetate, lauryl sulfate, gluconate, glutamate, hydroxynaphthoate, salicylate, stearate, trifluoroacetate (TFA), maleate, dimaleate, and muconate.

The ester of the compound represented by Formula (I) is not limited as long as it is a pharmaceutically acceptable ester, and examples of the ester include carbonate esters, phosphate esters, nitrate esters, sulfate esters, borate esters, and sulfonate esters. The present embodiment includes various hydrates, solvates, and crystalline polymorphs of the compound of Formula (I) and salts thereof.

The TREM2 signal activator may be selected using a cell made to express TREM2 and DAP12 such that binding of a ligand causes the TREM2 to generate a signal to allow expression of a reporter protein. The cell can be made to express TREM2 and DAP12 by a known method such as introduction of a gene expression plasmid into the cell. Any reporter protein can be used, and examples of the reporter protein include green fluorescent protein (GFP) and the luminescent protein luciferase. Signaling from TREM2 is known to cause expression of genes regulated by transcription factors of the "nuclear factor of activated T cells (NFAT)" type. Therefore, as the cell to be made to express TREM2 and DAP12, a cell containing an NFAT-type transcription factor may be preferably used. Specifically, the evaluation of the ligand may be carried out using a cell in which a gene encoding a reporter protein has been introduced as a gene whose expression is regulated by a NFAT type transcription factor, and in which TREM2 and DAP12 have been made to be expressed.

Hematopoietic progenitor cells are cells contained in bone marrow or cord blood. The hematopoietic progenitor cells are, for example, myeloid progenitor cells. Bone marrow cells collected from bone marrow, or mononuclear cells collected from umbilical cord blood may be used as the hematopoietic progenitor cells. The hematopoietic progenitor cells are especially preferably macrophage and dendritic cell progenitors (MDPs) or common monocyte progenitors (cMoPs)

In the culture step, the concentration of the hematopoietic progenitor cells is not limited. For example, the cells are plated at 1×10³ to 1×10⁷ cells or 1×10⁴ to 1×10⁶ cells per unit culture area (cm²). Preferably, 1×10⁵ hematopoietic progenitor cells are plated per unit culture area (cm²).

In the culture step, the hematopoietic progenitor cells may be cultured in a cell culture vessel that retains the TREM2 signal activator. Examples of the cell culture vessel include cell culture plates, cell culture flasks, and cell culture dishes. In cases where the TREM2 signal activator is insoluble in water, and hence insoluble in a liquid medium, the hematopoietic progenitor cells may be cultured in a cell culture vessel whose surface is coated with the TREM2 signal activator. In cases where a lipid derived from brain is used as the TREM2 signal activator, the mass of the lipid with which the surface of the cell culture vessel is to be coated is, for example, not less than 1.0 µg/cm², 1.5 to 20 µg/cm², 2 to 18 µg/cm², or 3 to 16 µg/cm², preferably 3 to 12 µg/cm², with respect to 1×10⁴ to 1×10⁶ hematopoietic progenitor cells. The coating of the surface of the cell culture vessel with the TREM2 signal activator may be carried out by dissolving (suspending) the ligand in an organic solvent or the like, adding the resulting solution (suspension) to the cell culture vessel, and then evaporating the organic solvent.

In the culture step, the cells may be cultured by a cell culture method using a known medium. The culture is carried out, for example, under conditions at 37°C at a CO₂ concentration of 5%. During the culture, the medium is preferably replaced, for example, every 3 to 4 days. About 7 days to 10 days after the beginning of the culture, macrophage-like adherent cells are obtained.

Macrophages obtained by the production method according to the present embodiment remain proliferative as long as they are stimulated with the TREM2 signal activator, and the number of days for which the macrophages can be cultured (survival period) is at least 540 days. Further, since the macrophages remain proliferative in culture after cryopreservation and thawing, they can be stored by cryopreservation. Further, since the macrophages stop proliferating when they are cultured in the absence of the TREM2 signal activator, the macrophages are not tumor cells. Therefore, the macrophages can be used in vivo without limitation, and are widely applicable as a research tool. The macrophages can be used also in cell therapy for diseases such as cancers.

Another embodiment provides a macrophage whose differentiation is induced dependently on TREM2 by culturing hematopoietic progenitor cells in a medium containing neither M-CSF nor GM-CSF, but containing a TREM2 signal activator.

As shown in Test Example 4 below, BMDMs induced from bone marrow cells with M-CSF lost proliferative capacity after 5 days of subculture in medium not containing M-CSF, while the above macrophage had proliferative capacity even after 10 days of subculture in a medium not containing M-CSF, in the presence of a TREM2 signal activator. Thus, another embodiment provides a macrophage having proliferative capacity after 10 days, preferably after 15 days, after 20 days, after 50 days, or after 100 days, more preferably after 200 days, after 300 days, or after 400 days, still more preferably after 500 days, of subculture in a medium containing neither M-CSF nor GM-CSF, but containing a TREM2 signal activator, after the beginning of induction or after cryopreservation.

As shown in Test Example 5 below, BMDMs thawed after cryopreservation did not proliferate even in the presence of M-CSF, while the above macrophage, after thawing, proliferated in the presence of a TREM2 signal activator. Thus, another embodiment provides a macrophage having, after cryopreservation, proliferative capacity in a medium containing neither M-CSF nor GM-CSF, but containing a TREM2 signal activator. The temperature for the cryopreservation is not limited as long as it is a temperature normally used for cryopreservation of cells, and may be, for example, -80°C. The period of the cryopreservation is not limited. The cryopreservation may be carried out until immediately after confirmation of freezing, may be carried out for 5 days, 10 days, 20 days, or 30 days after freezing, or may even be carried out for several months or several years after freezing.

Furthermore, as shown in Test Example 10 below, a culture supernatant of the macrophage after 24 hours of culture in a medium supplemented with LPS (at, for example, 10 ng/ml) contained MCP-1, TNF-α, IL-6, and NO at lower concentrations compared to those in a culture supernatant of BMDMs after 24 hours of culture in a medium supplemented with LPS. Thus, another embodiment provides a macrophage whose culture supernatant after 24 hours of culture in a medium supplemented with LPS contains TNF-α, IL-6, and nitric oxide at concentrations of not more than 30% as compared to BMDMs induced from bone marrow cells with M-CSF. The concentration of TNF-α in the culture supernatant of the macrophage may be not more than 25% or not more than 20% relative to the concentration in the culture supernatant of the BMDMs. The concentration of IL-6 in the culture supernatant of the macrophage may be not more than 25%, not more than 20%, or not more than 15% relative to the concentration in the culture supernatant of the BMDMs. The concentration of NO in the culture supernatant of the macrophage may be not more than 25% relative to the concentration in the culture supernatant of the BMDMs.

Further, expression of one or more, two or more, three or more, four or more, five or more, 10 or more, 20 or more, 30 or more, 40 or more, 50 or more, 60 or more, 70 or more, 80 or more, or the 88 genes listed in Table 3 may be higher in the macrophage according to the present embodiment than in at least one, preferably two or more, three or more, four or more, five or more, six or more, seven or more, or all eight selected from an alveolar macrophage, a BMDM, a BMDM induced to M1, a BMDM induced to M2, a Kupffer cell, a microglial cell, an osteoclast, and a peritoneal exudate macrophage.

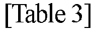

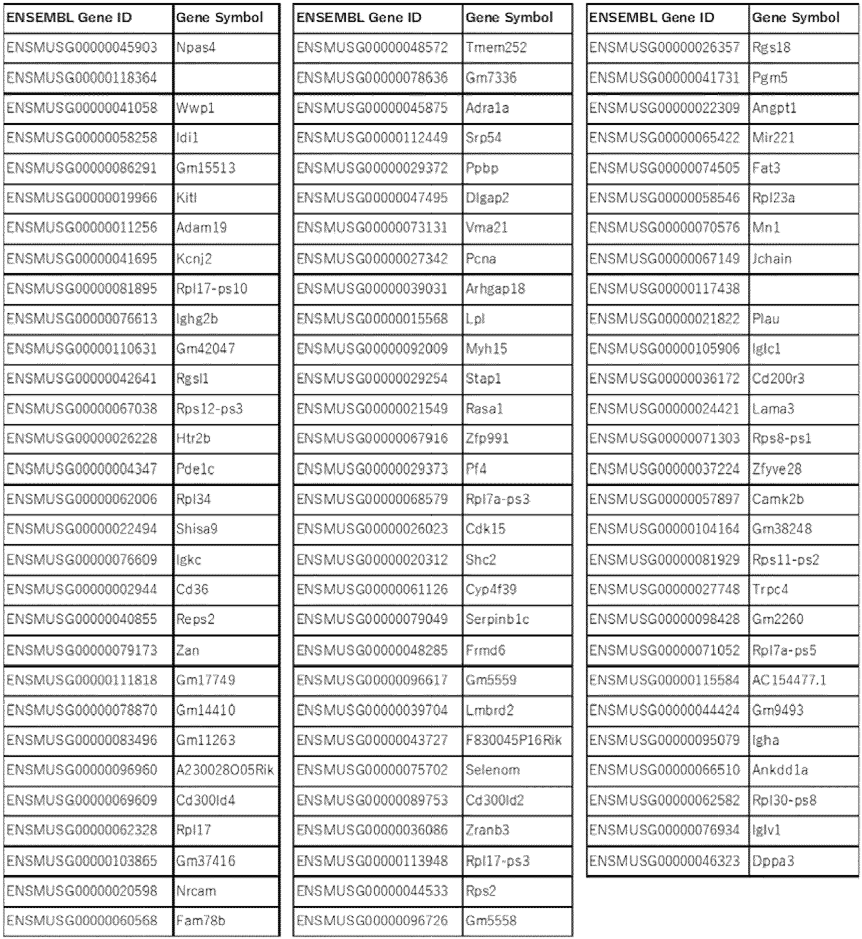

Irrespective of expression of the genes in Table 3, or in addition to high expression of the genes in Table 3, expression of one or more, two or more, three or more, four or more, five or more, 10 or more, 20 or more, 30 or more, 40 or more, 50 or more, 60 or more, 70 or more, 80 or more, or the 87 genes listed in Table 4 may be lower in the macrophage than in at least one, preferably two or more, three or more, four or more, five or more, six or more, seven or more, or all eight selected from an alveolar macrophage, a BMDM, a BMDM induced to M1, a BMDM induced to M2, a Kupffer cell, a microglial cell, an osteoclast, and a peritoneal exudate macrophage.

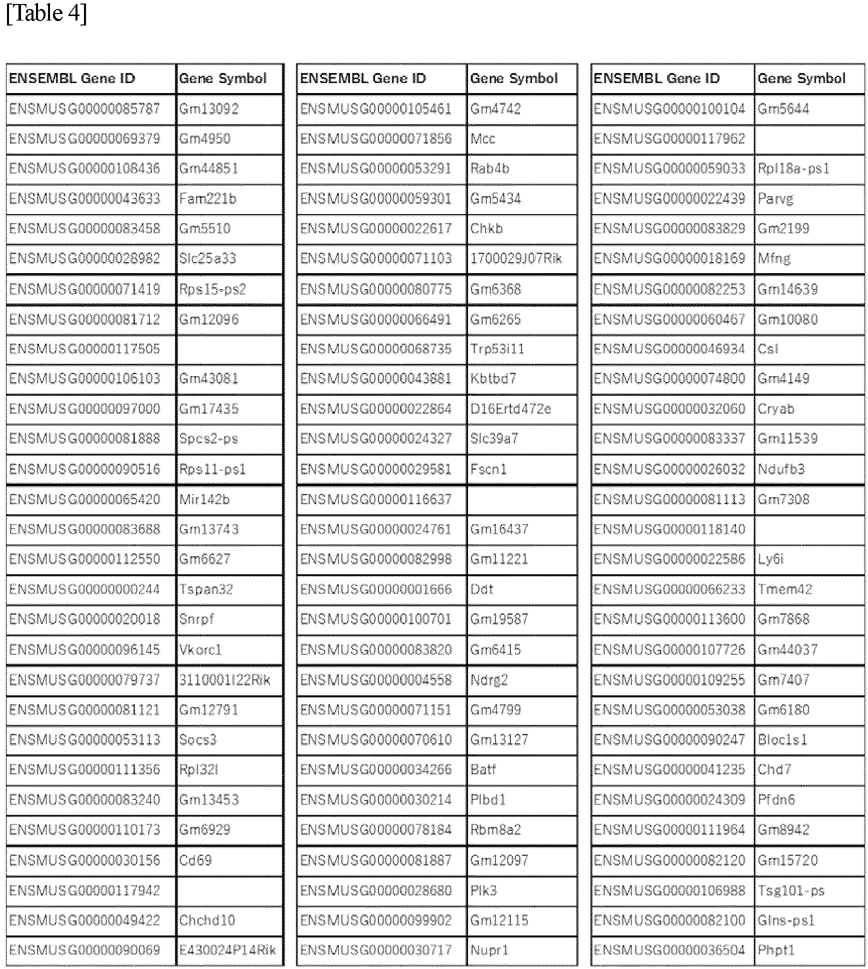

The expression of the above genes is preferably the RNA expression level. Throughout the present specification, the term "level" means an index of abundance expressed in a numerical value. It is, for example, the concentration, the amount, or an index that can be used as a substitute of the concentration or the amount. Thus, the level may be a measured value, or a value converted to the concentration. The level may be an absolute value such as the abundance or the abundance per unit area, or may be a relative value based on comparison with a comparative control that is set when necessary.

The macrophage according to the present embodiment may have any one, any two or more, any three or more, any four or more, any five or more, or all characteristics selected from the characteristics described above.

Another embodiment provides a method of preparing macrophages, a method of manufacturing macrophages, a method of inducing macrophages, a method of culturing macrophages, or a method of proliferating macrophages, including the culture step described above. By the method of culturing macrophages or the like, the macrophages can be maintained for a long period. By the method of proliferating macrophages, macrophages are proliferated. The method of producing macrophages, the method of preparing macrophages, the method of manufacturing macrophages, the method of inducing macrophages, the method of culturing macrophages, and the method of proliferating macrophages preferably use neither M-CSF nor GM-CSF.

Another embodiment provides an agent for differentiation induction including a TREM2 signal activator. The agent for differentiation induction induces differentiation from hematopoietic progenitor cells into macrophages. The agent for differentiation induction may include a solvent, water, ethanol, a polyol, an oil component, a surfactant, a thickener, an antiseptic, a pH-adjusting agent, or the like in addition to the TREAD signal activator. The agent for differentiation induction may be in the form of, for example, a liquid, a gel, a cream, or a solid. Preferably, the agent for differentiation induction is used in a state where the agent is added to a cell culture vessel, or where the agent is applied to the surface of a cell culture vessel to coat the surface of the vessel.

The agent for differentiation induction enables production of macrophages that have a long survival period and can be stored by cryopreservation, from hematopoietic progenitor cells. The macrophages are widely applicable as a research tool, and can be easily used in various experiments.

Another embodiment provides an agent for promoting proliferation of macrophages, the agent including a TREM2 signal activator. The agent for promoting proliferation promotes proliferation of macrophages, in particular, macrophages obtained by the production method described above. The agent for promoting proliferation may include components other than a TREM2 signal activator, similar to the agent for differentiation induction.

Another embodiment provides a kit for differentiation induction, including a cell culture vessel having a surface coated with a TREM2 signal activator. The kit for differentiation induction is used to induce differentiation from hematopoietic progenitor cells into macrophages. The kit for differentiation induction may also include a medium such as a basal medium, a low-serum medium, or a serum-free medium; a serum required for cell culture, such as fetal bovine serum (FBS); or another known additive to be added to a medium.

Since the cell culture vessel includes a cell culture vessel having a surface preliminarily coated with a TREM2 signal activator, macrophages that have a long survival period and can be stored by cryopreservation can be simply obtained merely by plating hematopoietic progenitor cells in the cell culture vessel and culturing those cells. The kit for differentiation induction can be used also as a kit for promoting proliferation of macrophages.

The agent for differentiation induction and the agent for promoting proliferation of macrophages described above preferably contain neither M-CSF nor GM-CSF. Further, the kit for differentiation induction into macrophages and the kit for promoting proliferation of macrophages described above preferably contain neither M-CSF nor GM-CSF.

### Examples

The present disclosure is described more concretely by way of the following Examples. However, the present disclosure is not limited by the Examples.

### Example 1: Preparation of Lipid-Induced Macrophages (LIMs)

### [Extraction of Mouse Brain Lipid]

Mouse brain (about 400 mg/mouse) was immersed in 1 ml of methanol in a polypropylene tube, and minced with scissors. The entire amount of the obtained sample was transferred to a glass test tube, and 1 ml of methanol and 4 ml of chloroform were added thereto to achieve a volume ratio of chloroform: methanol (MeOH) of 2:1 (C-M 2:1). The sample was mixed for 3 hours at room temperature using a shaker (200 rpm). The sample was centrifuged at 2000 rpm for 15 minutes, and then the supernatant was filtered and collected into a glass bottle, to obtain Extract 1.

To the residual brain in the glass test tube, 2 ml of methanol and 4 ml of chloroform were newly added, and the resulting sample was mixed for 16 hours at room temperature using a shaker (200 rpm). After filtration, the supernatant was collected into the glass bottle containing Extract 1, and the resulting mixture was mixed well, to obtain Extract 2. Extract 2 was stored at -30°C until use.

Into a weighed glass bottle, 1.8 ml of Extract 2 was dispensed, and the solvent was evaporated by blowing nitrogen under heat at 37°C, followed by measuring the dry weight of lipid. The lipid was dissolved in C-M 2:1 at a concentration of 5 mg/ml, to prepare a brain lipid extract.

### [Coating of Cell Culture Plate with Brain Lipid]

The brain lipid extract was diluted to 50 µg/ml with methanol, and dispensed into a cell culture plate at 3 µg/cm² The solvent was evaporated in a clean bench.

### [Induction of Lipid-Induced Macrophages]

Collected mouse bone marrow cells were suspended in 10% FBS/RPMI at 4×10⁵ cells/ml. The bone marrow cell suspension was added to the cell culture plate coated with the brain lipid, at 1×10⁵ cells/0.25 ml/cm². The bone marrow cells were cultured at 37°C in a 5% CO₂ incubator. On Day 3 of the culture, fresh medium in a half amount was added to the culture plate. Thereafter, the medium was replaced every 3 to 4 days.

### (Results)

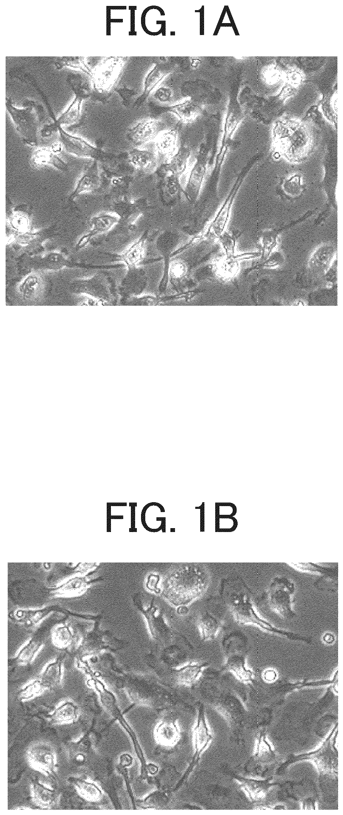

About 7 days to 10 days after the beginning of the culture, macrophage-like adherent cells have begun to appear. Panel (A) of FIG. 1 is an image of LIMs on Day 14 after the beginning of the culture.

### Comparative Example 1: Preparation of BMDMs

Collected mouse bone marrow cells were suspended in 10% FBS/RPMI at 4×10⁵ cells/ml. To the resulting cell suspension, 25 ng/ml M-CSF was added. To each well of a 24-well culture plate, the bone marrow cell suspension was added at 2×10⁵ cells/0.5 ml. The bone marrow cells were cultured at 37°C in a 5% CO₂ incubator. On Day 3 of the culture, 25 ng/ml M-CSF/10% FBS/RPMI in a half amount (0.25 ml/well) was added. On Day 5 of the culture, the supernatant was aspirated, and 0.5 ml/well of 10% FB S/RPMI was added, followed by detaching the cells using a scraper. After 4-fold dilution with 10% FBS/RPMI, the resulting dilution was dispensed at 0.5 ml/well, and 25 ng/ml M-CSF was added thereto. Thereafter, half the medium was replaced with 25 ng/ml M-CSF/10% FBS/RPMI every 3 to 4 days.

### (Results)

On Day 5 after the beginning of the culture, the cells increased to 4.0 to 6.5×10⁵ cells/well. Panel (B) of FIG. 1 is an image of BMDMs induced with M-CSF, on Day 5 after the beginning of the culture.

### Test Example 1: Identification of Cell Type

### [Total RNA Extraction from LIMs]

Total RNA was extracted from LIMs as follows according to the manufacturer's instructions for Sepasol (trademark)-RNAI Super G (manufactured by Nacalai Tesque, Inc.). After adding 1 ml of Sepasol (trademark)-RNA I Super G to LIMs (1.6×10⁶ cells) on Day 14 after the beginning of the culture, the resulting mixture was homogenized by pipetting. The sample was stirred using a vortex mixer, and then left to stand at room temperature for 5 minutes. After adding 200 µl of chloroform to the sample, the resulting mixture was mixed by inversion, and then left to stand at room temperature for 3 minutes. The sample was centrifuged at 12,000 G at 4°C for 15 minutes, and then the aqueous phase, corresponding to the upper layer, was transferred to a 1.5-ml tube. To the aqueous phase, 500 µl of isopropanol was added, and the resulting mixture was mixed, followed by leaving the sample to stand at room temperature for 10 minutes. Subsequently, the sample was centrifuged at 12,000 G at 4°C for 10 minutes, and then the supernatant was discarded. To wash the precipitate, 1 ml of 75% ethanol was added to the precipitate. Centrifugation was carried out at 12,000 G at 4°C for 5 minutes, and then the supernatant was discarded. After opening the lid of the tube, the tube was left to stand at room temperature for 10 minutes to evaporate the residual liquid, to obtain total RNA. The total RNA was dissolved by addition of 40 µl of nuclease-free water thereto.

### [DNase I Treatment of Total RNA].

The total RNA was treated with DNase I as follows according to the manufacturer's instructions for Recombinant DNase I (manufactured by Takara Bio Inc.). To 38 µl of the total RNA, 5 µl of 10×DNase buffer, 2 µl (10 U) of recombinant DNase I (RNase-free), 1 µl (20 U) of RNase inhibitor, and 4 µl of DEPC-treated water were added. The reaction was allowed to proceed at 37°C for 20 minutes, and 150 µl of DEPC-treated water was added to the reaction solution, to provide a sample with a total volume of 200 µl. Phenol/chloroform/isoamyl alcohol (at a volume ratio of 25:24:1) in an amount of 200 µl was added to the sample, and the resulting mixture was mixed together. The sample was then centrifuged at 12,000 G at room temperature for 5 minutes. About 200 µl of the aqueous phase was collected into a 1.5-ml tube, and 20 µl of 3M sodium acetate and 500 µl of ethanol were added to the tube, followed by leaving the mixed sample to stand at room temperature for 15 minutes.

The sample was centrifuged at 20,000 G at 4°C for 20 minutes, and then the supernatant was discarded. To wash the precipitate, 1 ml of 70% ethanol was added. The sample was centrifuged at 20,000 G at 4°C for 10 minutes, and then the supernatant was discarded. After opening the lid of the tube, the tube was left to stand at room temperature for 10 minutes to evaporate the residual liquid. RNA was dissolved by addition of 40 µl of TE buffer, and the concentration was measured using NanoDrop. After adjusting the RNA concentration to 150 ng/µl, the sample was stored at -80°C.

### [RNA-seq Analysis]

A next-generation sequencing analysis service by Veritas Genetics was used (service name: mRNA-seq (150 bp PE), data volume: 3 G, number of reads: 10 million reads) to obtain data in the fastq format.

### [RNA-seq Data Analysis]

By the pigz command, the acquired data were compressed into the fastq.gz format. By the fastp command, the adapter sequence was trimmed, and quality check was performed. By the hisat2 command, mapping on a reference genome (mouse: GRCm38_genome) was performed. By the samtools command, the mapping results were sorted and converted to a BAM file. By the featurecounts command, the reads were counted for each gene, to quantify the expression level. From the obtained read count data, the cell type was identified by reference to ImmGen database (https://www.immgen.org/) using the Single R pipeline.

### (Results)

By the RNA-seq data analysis, the cell type of the LIMs was identified as "macrophages".

### Test Example 2: Expression Analysis of Cell Surface Markers of BMDMs and LIMs

LIMs on Day 14 after the beginning of the induction in Example 1 and BMDMs on Day 5 after the beginning of the induction in Comparative Example 1 were separately collected by detaching the cells together with the medium using a cell scraper, and the cells were counted using a hemocytometer. Centrifugation was carried out at 500 G at 4°C for 5 minutes, and then the supernatant was discarded. 1% FBS/HBSS was added to the cells to prepare a suspension at 1×10⁷ cells/ml. An anti-mouse CD16/32 antibody (manufactured by BioLegend) was added to the cell suspension to 10 µg/ml, and the resulting mixture was left to stand on ice for 10 minutes, to perform blocking of Fcy receptors.

The cell suspension after the blocking was dispensed into a 96-well V-bottom plate at 10 µl/well, and a PE anti-mouse TREM2 antibody (manufactured by R&D), a PE anti-mouse Dectin-1 antibody, an FITC anti-mouse CD9 antibody, an APC anti-mouse CD11c antibody, a PE anti-mouse Siglec-F antibody, a PE anti-mouse CD206 antibody, an APC anti-mouse P2RY12 antibody, an APC anti-mouse CX3CR1 antibody, an APC anti-mouse MHC class II antibody, an APC/Cyanine7 anti-mouse CD115 antibody, a PE/Cyanine7 anti-mouse CD115 antibody, an FITC anti-mouse F4/80 antibody, a PE/Cyanine7 anti-mouse Ly-6C antibody, an FITC anti-mouse Ly-6G antibody, an APC anti-mouse CD45 antibody, or a PE anti-mouse CD11b antibody (all of these are manufactured by BioLegend) diluted with 1%FBS/HBSS was added to the cell suspension such that the recommended concentration described in the manufacturer's instructions was achieved in a total volume of 20 µl/well, followed by leaving the resulting mixture to stand on ice for 15 minutes to allow the reaction to proceed. To wash the sample, 180 µl/well of 1% FBS/HBSS was added.

The sample was centrifuged at 500 G at 4°C for 5 minutes, and then the supernatant was discarded. To wash the sample, 180 µl/well of 1% FBS/HBSS was added. The sample was centrifuged at 500 G at 4°C for 5 minutes, and then the supernatant was discarded. The sample was suspended by adding 50 µl/well of 10 µg/ml PI/1% FBS/HBSS. The obtained sample was analyzed by flow cytometry. In univariate histogram analysis of surface antigens, Ly6G-negative CD11b-positive cells were extracted as macrophage cells, and subjected to analysis of each surface antigen. For the detection of CD115, anAPC/Cyanine7 anti-mouse CD115 antibody was used in dot plot analysis by flow cytometry, and a PE/Cyanine7 anti-mouse CD115 antibody was used in univariate histogram analysis of surface antigens.

### (Results)

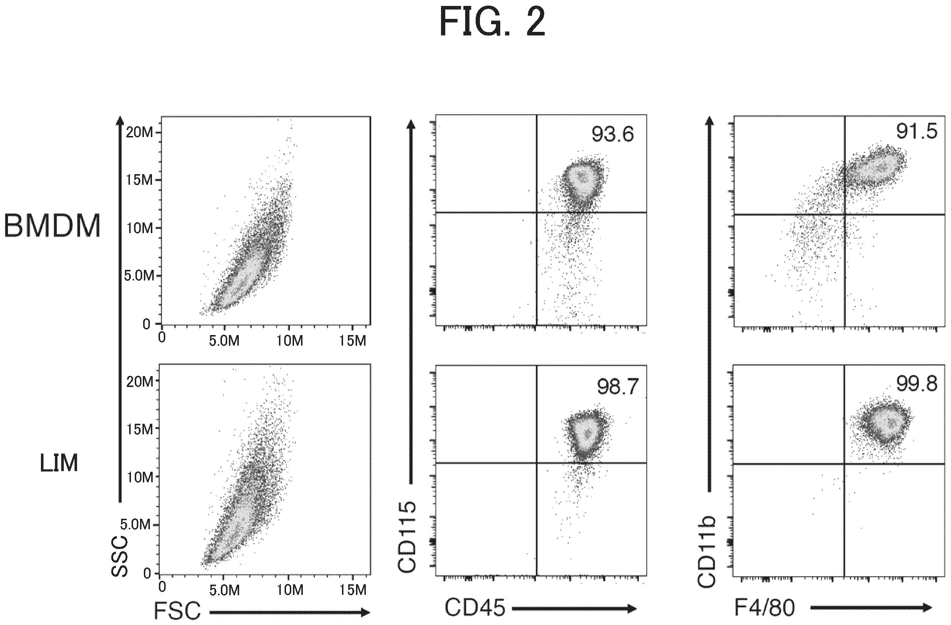

The results of the dot plot analysis are illustrated in FIG. 2. The LIMs and the BMDMs had similar signal distributions regarding the forward scatter (FSC), which is an index of the cell size, and the side scatter (SSC), which is an index of the complexity of cells (number of granules). The LIMs showed high expression of CD45, which is a pan-leukocyte marker, and CD115, which is a monocyte marker, and also showed high expression of F4/80, which is a macrophage marker, and CD1 1b, which is a marker of granulocytes and macrophages.

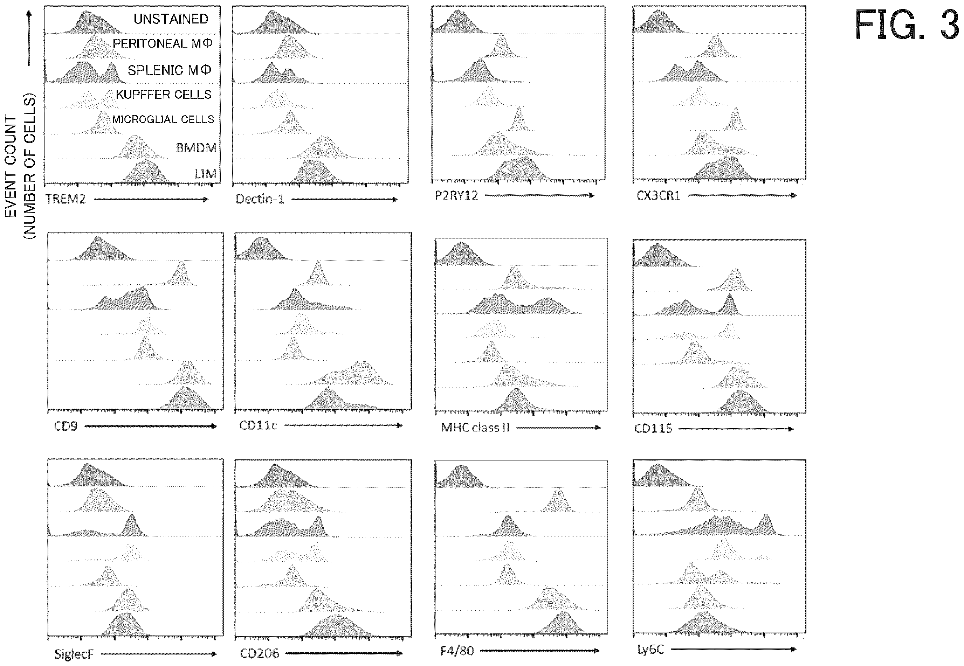

The results of the univariate histogram analysis of the surface antigens are illustrated in FIG. 3. The BMDMs and the LIMs showed very similar expression patterns of the surface antigens to each other when compared to macrophages in the body of mice, such as peritoneal macrophages (MΦ), splenic macrophages, Kupffer cells, and microglial cells. The expression levels of TREM2, CD206, P2RY12, and CX3CR1 were especially high in the LIMs. From the results of Test Example 1 and Test Example 2, the LIMs were shown to be macrophages.

### Test Example 3: Study of Lipid Concentration for LIM Induction

### [Coating with Brain Lipid]

With a brain lipid extract prepared in the same manner as in Example 1, a 96-well cell culture plate was coated at 4, 2, 1, 0.5, 0.25, or 0.125 µg/well. Collected mouse bone marrow cells were suspended in 10% FBS/RPMI at 4×10⁵ cells/ml. The bone marrow cell suspension was dispensed at 100 µl/well, and the cells were cultured at 37°C in a 5% CO₂ incubator. On Day 3 of the culture, fresh medium in a half amount was added. Thereafter, the medium was replaced every 3 to 4 days. On Day 7 and Day 14 after the beginning of the culture, the number of viable cells was measured by WST-8 assay.

### [WST-8 Assay]

To fresh 10% FBS/RPMI medium, 1/10 volume of WST-8 (07553-15 Cell Count Reagent SF, manufactured by Nacalai Tesque Inc.) was added to prepare a medium for counting the number of viable cells. The old medium in the culture plate was removed by aspiration, and the medium for counting the number of viable cells was dispensed at 100 µl/well. The cells were cultured at 37°C in a 5% CO₂ incubator for 1 hour. The absorbance at 450 nm was measured using a microplate reader.

### (Results)

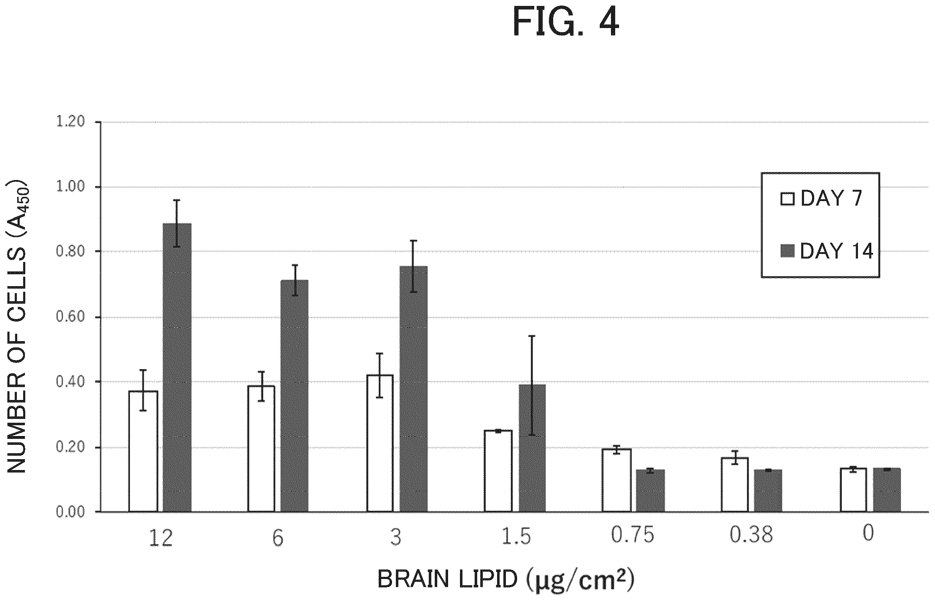

FIG. 4 illustrates the number of cells with respect to the mass of the brain lipid with which the culture plate was coated, per unit culture area. LIMs were suitably induced when the brain lipid was at not less than 1.5 µg/cm² In particular, proliferation of LIMs was promoted at 3 to 12 µg/cm².

### Test Example 4: Subculture of LIMs

In cases where a 24-well plate is used, the cells reach 90 to 100% confluence about 14 days after the beginning of the induction. Therefore, LIMs or BMDMs were subcultured every 14 days as follows. After aspiration of the culture supernatant, 0.5 ml/well of 1 mM EDTA/PBS was added, and the plate was incubated at 37°C for 5 minutes. After removing the 1mM EDTA/PBS by aspiration, 0.5 ml/well of 10% FBS/RPMI was added, and the cells were detached and suspended by pipetting. The number of cells was counted using a hemocytometer. The cell suspension was diluted 4-fold with 10% FBS/RPMI, and 0.5 ml/well of the resulting dilution was dispensed into a new brain lipid-coated plate. Thereafter, half of the 10% FBS/RPMI medium was replaced every 3 to 4 days, and the cells were subcultured again on Day 14.

In addition, LIMs cultured for 586 days were plated at 20,000 cells/well in a 24-well plate coated (BL+) or not coated (BL-) with brain lipid (3 µg/cm² of brain lipid, when coated), and then culture was performed.

### (Results)

In FIG. 5, the top row of Panel (A) illustrates the number of cells on Days 0 to 21 of the culture, and the bottom row of Panel (A) illustrates the fold increases in the number of cells during Days 0 to 21 of the culture relative to the number of cells on Day 0, which was taken as 1. In FIG. 5, the top row of Panel (B) illustrates the numbers of cells on Days 0 to 544 of the culture, and the bottom row of Panel (B) illustrates the fold increases in the number of cells during Days 0 to 544 of the culture relative to the number of cells on Day 0, which was taken as 1. The BMDMs hardly increased after the dilution by subculture. In contrast, the LIMs survived and proliferated for more than 544 days in the culture.

Panel (C) of FIG. 5 illustrates the number of cells of the LIMs cultured with BL+ or BL-. The LIMs with BL- did not proliferate and survive. Since the LIMs did not proliferate and survive without stimulation by the brain lipid, the LIMs were shown not to be neoplastic lymphocytes.

### Test Example 5: Cryopreservation Test of LIMs and BMDMs

LIMs and BMDMs at 80 to 90% confluence were detached from culture plates, and separately centrifuged at 500 G for 5 minutes, followed by discarding the supernatant. In a CELLBANKER 1 (manufactured by Nippon Zenyaku Kogyo Co., Ltd.), the cells were suspended at 5×10⁵ to 5×10⁶ cells per 1 ml, and the resulting suspension was transferred to a CryoTube, and stored at -80°C. Thirty days later, the cells were thawed, and plated in a 24-well plate without stimulation (None), with addition of 25 ng/ml M-CSF (M-CSF), or with brain lipid coating (BL). The number of viable cells on Day 6 or Day 14 after the plating was counted using a hemocytometer.

### (Results)

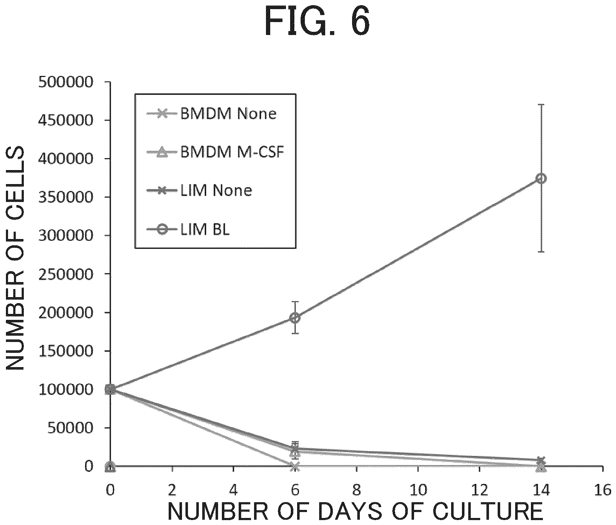

As illustrated in FIG. 6, the BMDMs thawed after the cryopreservation did not proliferate even in the presence of M-CSF. In contrast, the thawed LIMs proliferated in the presence of the stimulation with the brain lipid. Cryopreservation of the LIMs was thus shown to be possible.

### Test Example 6: Isolation of Cells by Magnetic Cell Sorting and Cell Sorter

To bone marrow cells of a CD45.1-positive mouse or a CD45.2-positive mouse, 10 µg/ml anti-Fcy receptor (CD16/32) antibody/0.5% BSA/PBS was added in an amount of 100 µl per 1×10⁷ cells, and then the cells were suspended. The resulting suspension was left to stand on ice for 5 minutes, to block Fcγ receptors. To the suspension of the bone marrow cells of each mouse, fluorescently labeled antibodies against the following mature cell markers (lineage marker: Lin) were added, and the reaction was allowed to proceed.
Anti-CD3-FITC (2.5 µg/ml)
Anti-CD19-FITC (2.5 µg/ml)
Anti-NK1.1-FITC (2.5 µg/ml)
Anti-Ly6G-FITC (2.5 µg/ml)
Anti-TER-119-FITC (2.5 µg/ml)
Anti-CD11b-FITC (2.5 µg/ml)

The bone marrow cell suspension was left to stand on ice for 15 minutes, and then subjected to operation according to the manufacturer's instructions for Anti-FITC MicroBeads and Anti-APC MicroBeads, manufactured by Miltenyi Biotec. The sample was washed by addition of MACS (trademark) buffer (0.5% BSA/2mM EDTA/PBS) in an amount of 1 ml per 1×10⁷ cells, and then centrifuged at 300 G at 4°C for 10 minutes, followed by discarding the supernatant. The precipitate was suspended in 80 µl of MACS (trademark) buffer, and 20 µl of anti-FITC antibody-labeled magnetic beads were added thereto. The sample was left to stand at 4°C for 15 minutes, and then washed by addition of 1 ml of MACS (trademark) buffer thereto. The sample was centrifuged at 300 G at 4°C for 10 minutes, and then the supernatant was discarded. The precipitate was suspended in 0.5 ml of MACS (trademark) buffer. An LS column was placed in a MACS (trademark) separator, and washed with 3 ml of MACS (trademark) buffer in advance. The sample was added to the LS column, and the passthrough fraction was collected as a Lin-negative fraction.

Washing of the LS column with 3 ml of MACS (trademark) buffer was repeated three times. The LS column was removed from the MACS (trademark) separator, and placed on a 15-ml tube. After adding 5 ml of MACS (trademark) buffer to the LS column, the buffer was extruded using a plunger, to collect a Lin-positive fraction. Each of the Lin-negative fraction derived from the CD45.2-positive mouse and the Lin-positive fraction derived from the CD45.1-positive mouse was centrifuged at 500 G at 4°C for 10 minutes, and then supernatant was discarded. The obtained cells were suspended in 10% FBS/RPMI at 5×10⁶ cells/ml, and then the Lin-negative fraction and the Lin-positive fraction were strictly isolated by a cell sorter. The sample containing the isolated cells was centrifuged at 500 G at 4°C for 10 minutes, and then the supernatant was discarded. The obtained cells were suspended in 10% FBS/RPMI at 4×10⁵ cells/ml such that the Lin-negative fraction derived from the CD45.2-positive mouse and the Lin-positive fraction derived from the CD45.1-positive mouse were mixed at a ratio of 1:9, which is almost the same ratio as in the bone marrow. The resulting suspension was plated on a culture plate coated with the brain lipid. In addition, each fraction alone was similarly plated. In order to evaluate whether the Lin-positive cells or the Lin-negative cells are induced to differentiate into LIMs by stimulation with the brain lipid, the number of CD11b-positive F4/80-positive macrophages was counted by flow cytometry on Days 5, 9, and 14 after the beginning of the culture.

### (Results)

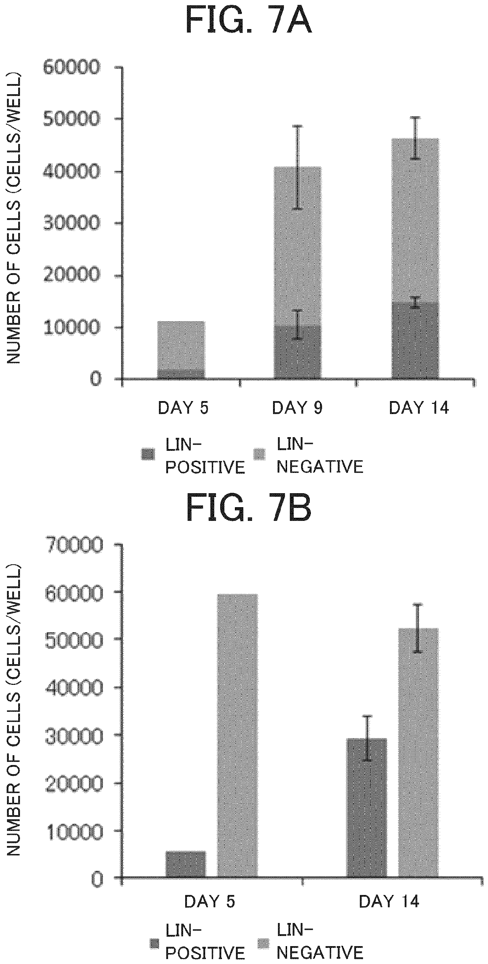

Changes in the number of macrophages that have differentiated from the Lin-positive cells derived from the CD45.1-positive mouse or the Lin-negative cells derived from the CD45.2-positive mouse in the mixed culture, and changes in the number of macrophages in the culture of each fraction alone, are illustrated in Panel (A) and Panel (B) of FIG. 7, respectively. Although the ratio between the Lin-negative cells and the Lin-positive cells at the beginning of the culture was 1:9, the majority (66 to 83%) of the cells induced to differentiate into LIMs were Lin-negative cells. Furthermore, in the case where the Lin-negative cells were cultured alone, differentiation induction into a large number of LIMs was found in an early period (on Day 5). These findings suggest that LIMs differentiate mainly from myeloid progenitor cells. Further, since a certain number of macrophages were induced also from Lin-positive cells, it was suggested that their differentiation may occur also from CD11b-positive monocytes.

### Test Example 7: Induction of Bone Marrow Cells Derived from Each Mouse into BMDMs and LIMs

In the same manner as in Example 1 and Comparative Example 1, LIMs and BMDMs were induced using bone marrow cells derived from a wild-type (WT) mouse (C57BL/6), a TREM2 knockout mouse (KO), or a mouse in which TREM2 R47H mutation was introduced. The TREM2 R47H mutation results in decreased ligand-binding capacity compared to the wild type. The LIMs were subjected to WST-8 assay on Day 14 after the beginning of the induction.

### (Results)

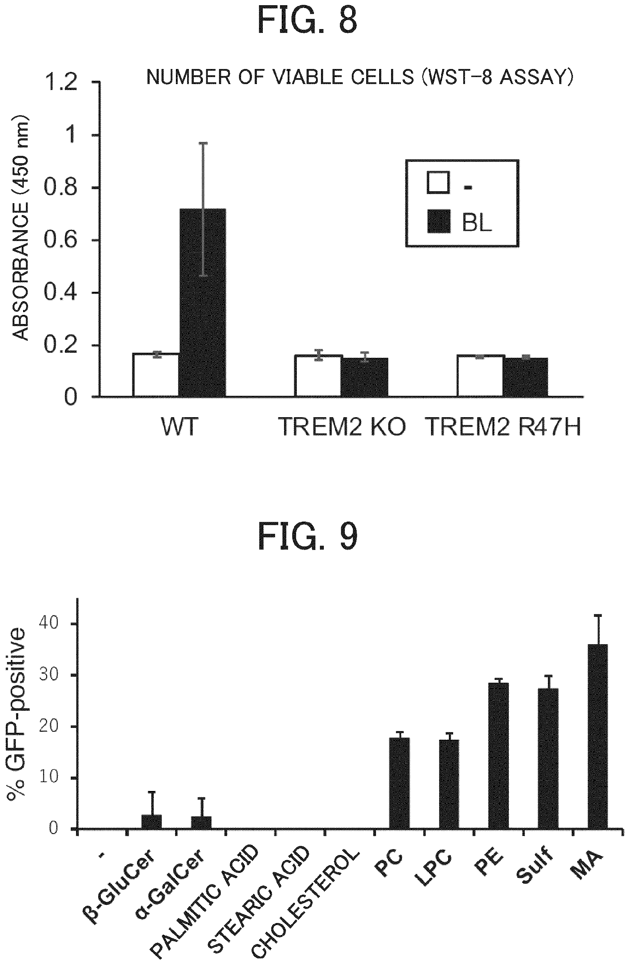

The BMDMs were observed under a microscope on Day 5 after the beginning of the induction, to confirm that they can be induced from cells derived from any of the mice. FIG. 8 illustrates the number of viable cells based on the evaluation by the WST-8 assay. The bone marrow cells derived from the wild-type mouse could be induced to differentiate into LIMs by the stimulation with the brain lipid. In contrast, the bone marrow cells derived from TREM2 KO or TREM2 R47H could not be induced to differentiate into LIMs even by the stimulation with the brain lipid.

### Test Example 8: Evaluation of Ligand Activity Using TREM2 Reporter Cells

Using a retroviral vector, TREM2 and DAP12 cDNAs were introduced into 2B4-NFAT-GFP reporter cells (obtained from RIKEN), to prepare reporter cells in which binding of a ligand to TREM2 expressed on the cell surface induces expression of GFP (Iizasa and others, Nature Communications, 2021, vol. 12(1), p. 2299-16).

As a test substance, β-GluCer, α-GalCer, palmitic acid, stearic acid, cholesterol, PC, LPC, PE, Sulf, or MA was diluted with methanol to 50 µg/ml. The test substance was aliquoted in 20-µl (1-µg) portions into a 96-well cell culture plate, and then the plate was left to stand in a clean bench, to evaporate the methanol.

In the culture plate coated with the test substance, the reporter cells were plated at 5×10⁴ cells/100 µl/well. The cells were cultured in a CO₂ incubator for 16 hours. The culture was suspended well, and transferred to a V-bottom 96-well plate. The sample was centrifuged at 500 G at 4°C for 5 minutes, and then the supernatant was discarded. The sample was suspended by adding 50 µl/well of 10 µg/ml PI/1% FBS/HBSS. GFP-positive cells in the sample were analyzed by flow cytometry.

A 96-well cell culture plate was coated with each test substance in the same manner as described above, and mouse bone marrow cells were plated in the plate at 4×10⁴ cells/100 µl/well. The cells were cultured in a CO₂ incubator, and WST-8 assay was carried out on Days 5, 10, 14, and 20 after the beginning of the culture, to count the number of viable cells.

### (Results)

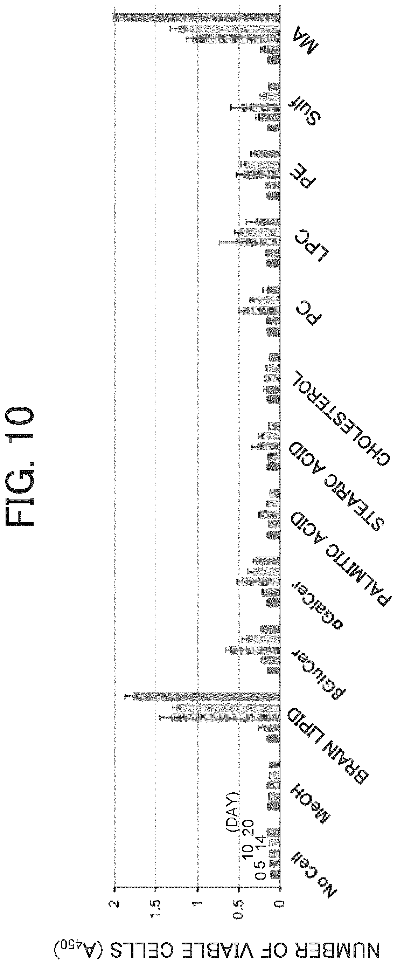

As illustrated in FIG. 9, β-GluCer, α-GalCer, PC, LPC, PE, Sulf, and MAwere found to have ligand activity for TREM2. β-GluCer, α-galactosylceramide, PC, LPC, PE, Sulf, and MA induced differentiation of the bone marrow cells into LIMs as illustrated in FIG. 10.

### Test Example 9: Evaluation of Phagocytotic Ability

BMDMs or LIMs were plated in a 96-well plate at 1×10⁵ cells/200 µl/well, and 1×10⁶ cfu of FITC-labeled bacille Calmette-Guerin (BCG) was added thereto, followed by incubation at 37°C for 4 hours. Four hours later, the cells were collected, and the fluorescence intensity of FITC was measured by flow cytometry, to compare the phagocytotic ability of the FITC-labeled BCG (BCG-FITC).

### (Results)

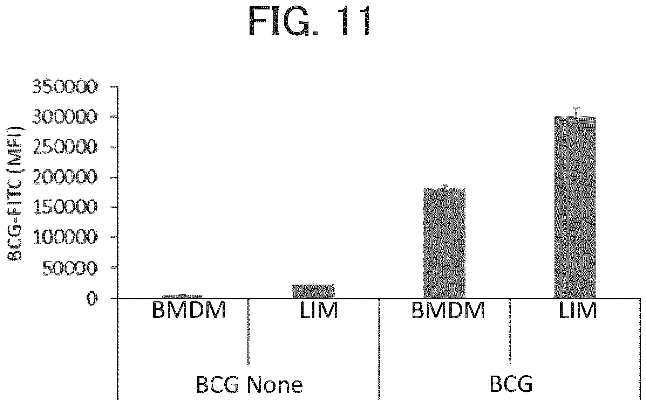

FIG. 11 illustrates the mean fluorescence intensity (MFI) associated with the FITC-labeled BCG. The LIMs were found to have higher phagocytotic ability compared to the BMDMs.

### Test Example 10: Study of Cytokine Production Response to LPS Stimulation

BMDMs or LIMs were plated in a 96-well plate at 1×10⁵ cells/200 µl/well, and 10 ng/ml LPS was added thereto, followed by incubation at 37°C for 24 hours. The culture supernatant was collected 24 hours later, and the concentrations of MCP-1, TNF-α, IL-6, IL-10, and NO in the supernatant were measured.

### (Results)

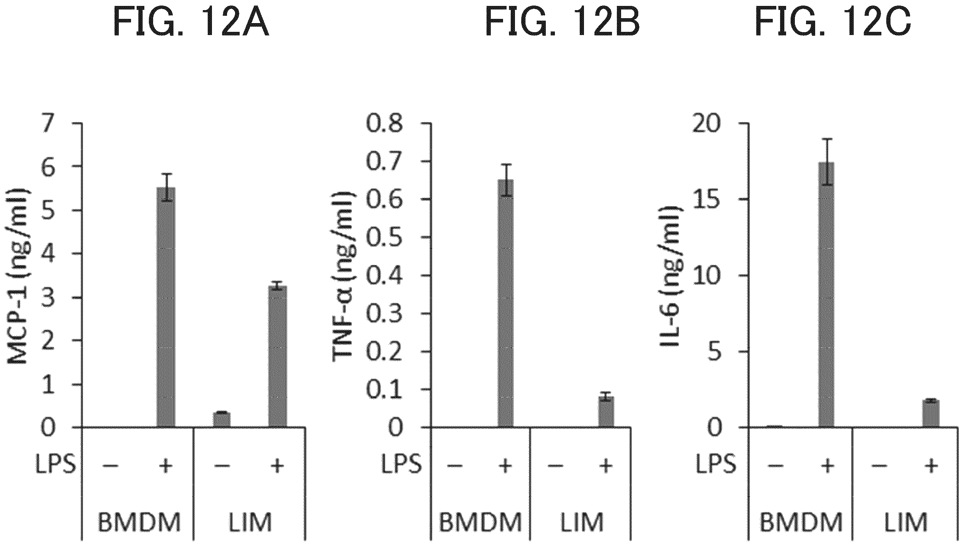

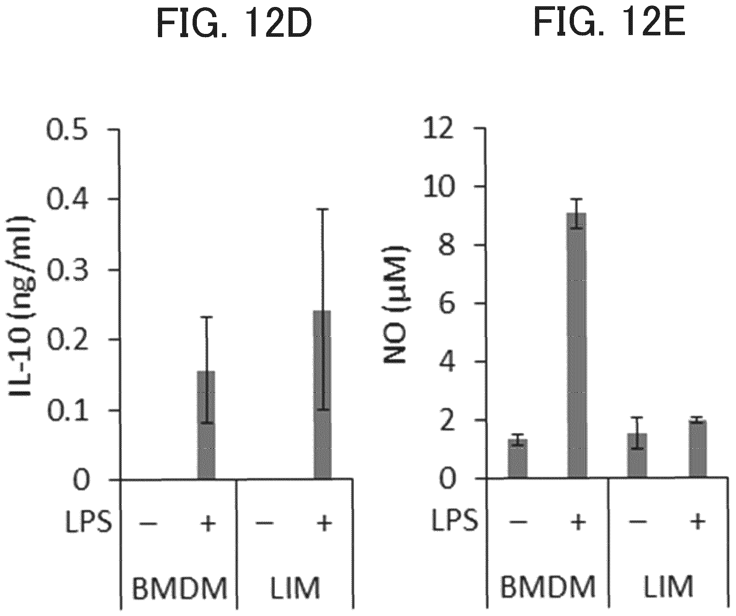

Panels (A), (B), (C), (D), and (E) of FIG. 12 illustrate the concentrations of MCP-1, TNF-α, IL-6, IL-10, and NO, respectively. Compared to the BMDMs, the LIMs showed lower production of inflammatory cytokines such as IL-6 and TNF-α; and NO.

### Test Example 11: Study of Marker Molecules for LIMs

RNA-seq data (SRA files) for various cells were obtained from the Gene Expression Omnibus (GEO) database (https://www.ncbi.nlm.nih.gov/geo/). The reference sources (GEO accession number) of the cells are as follows.
Alveolar macrophages: GSM4476109, GSM4476110, and GSM4476111
BMDMs: GSM4552500, GSM4552501, and GSM4552502
BMDMs (M1): GSM4844180, GSM4844181, and GSM4844182
BMDMs (M2): GSM4844183, GSM4844184, and GSM4844185
Kupffer cells: GSM4119149, GSM4119150, and GSM4119151
Microglial cells: GSM4065894, GSM4065895, and GSM4065896
Osteoclasts: GSM3179449, GSM3179450, and GSM3179451
Peritoneal exudate macrophages: GSM2859803, GSM2859804, and GSM2859805

The BMDMs (M1) are cells obtained by adding 100 ng/ml LPS and 50 ng/ml mlFNy to BMDMs, and culturing the cells for 24 hours. The BMDMs (M2) are cells obtained by adding 20 ng/ml IL-4 and 20 ng/ml IL-13 to BMDMs, and culturing the cells for 24 hours.

By the fasterq-dump command, the SRA files for the various cells were converted to fastq-format data. Read count data were obtained according to the method described for the analysis of RNA-seq data in Test Example 1 above. Using the read count data of LIMs and these cells, differentially expressed genes (DEGs) were extracted by DESeq2. Further, based on the lists of the DEGs, the LIMs and all other macrophages were compared to extract DEGs that consistently show high expression in the LIMs (88 DEGs) and DEGs that consistently show low expression in the LIMs (87 DEGs), as marker molecules. The extraction was carried out using Calculate and draw custom Venn diagrams (http://bioinformatics.psb.ugent.be/webtools/Venn/).

### (Results)

Table 5 illustrates the number of the DEGs. The DEGs showing high expression in the LIMs and the DEGs showing low expression in the LIMs extracted are listed in Table 6 and Table 7, respectively.

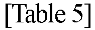

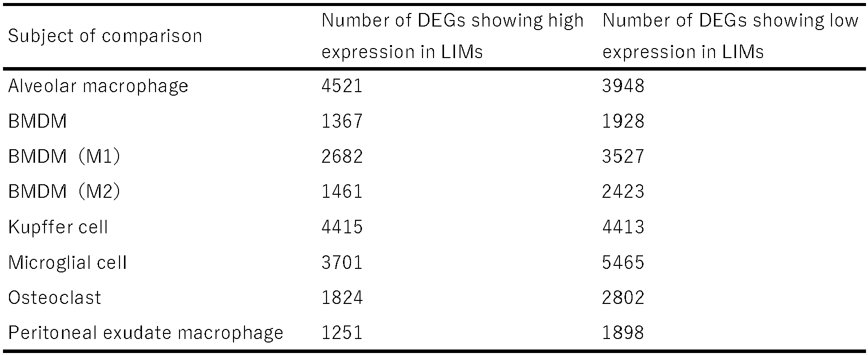

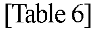

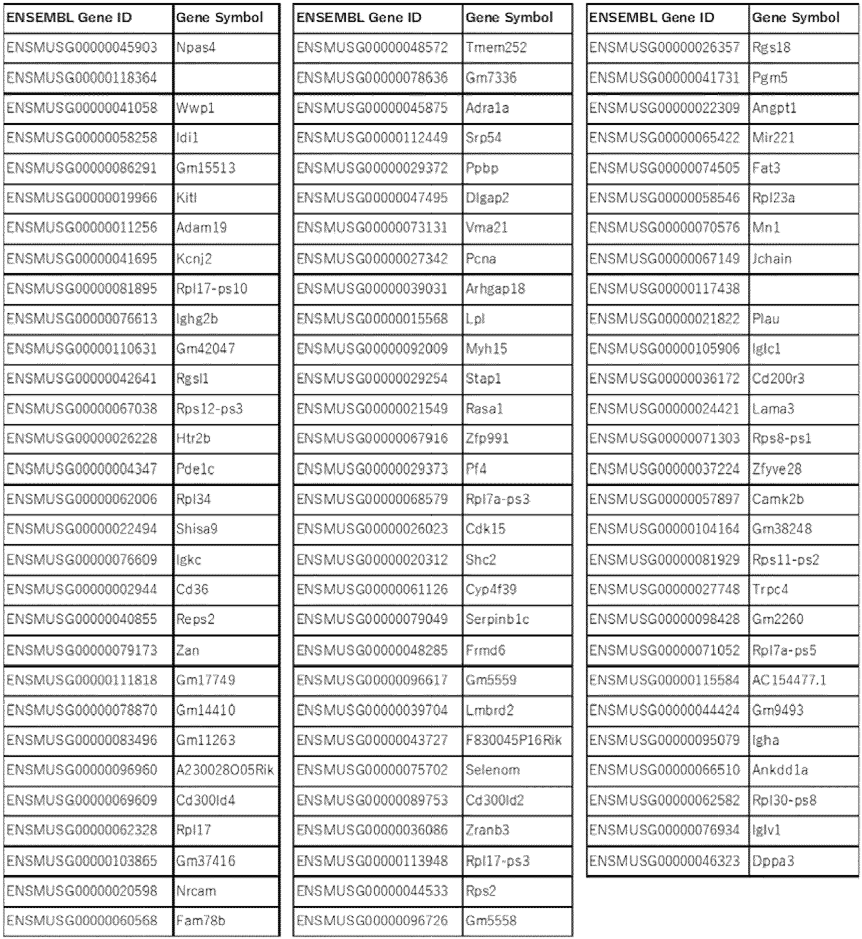

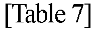

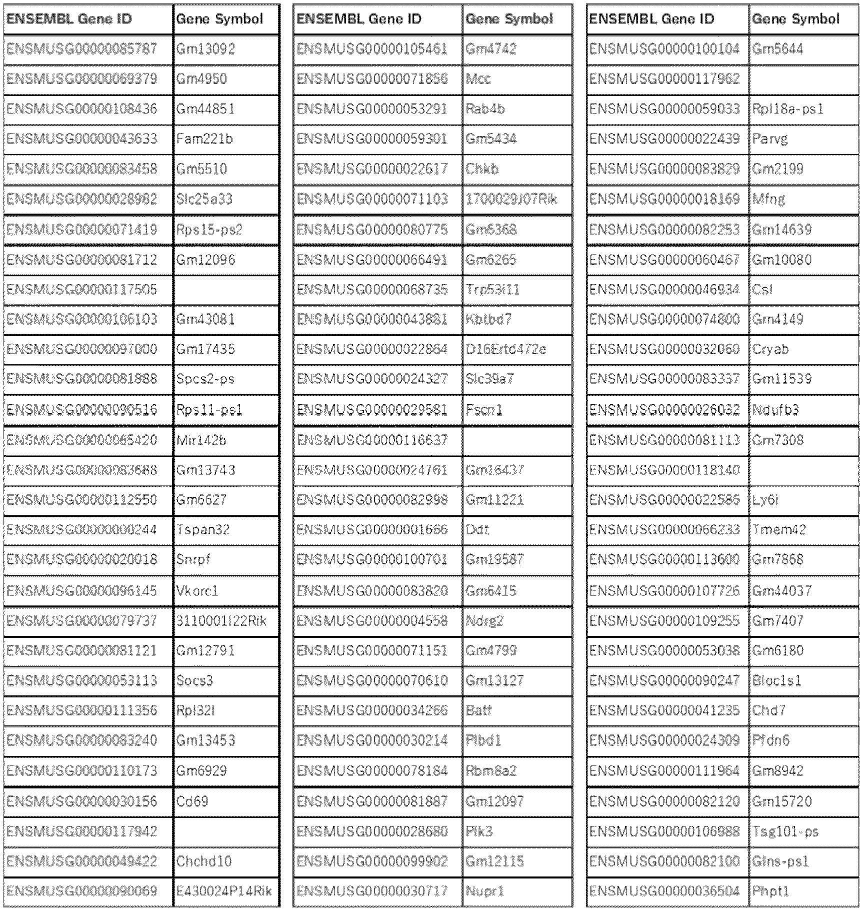

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2021-208420, filed on December 22, 2021, the entire disclosure of which is incorporated by reference herein.

### Industrial Applicability

The present disclosure is useful for obtaining macrophages.

## Claims

1. A method of producing macrophages, the method comprising:
a culture step of culturing hematopoietic progenitor cells in presence of a TREM2 signal activator.

2. The method of producing macrophages according to claim 1, wherein
the TREM2 signal activator is a lipid.

3. The method of producing macrophages according to claim 1 or 2, wherein
the TREM2 signal activator is a lipid derived from brain or a lipid containing two long-chain fatty groups.

4. The method of producing macrophages according to claim 1 or 2, wherein
the TREM2 signal activator is a compound represented by Formula (I): 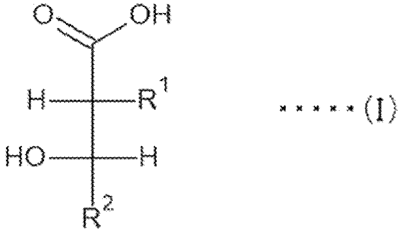
(wherein the compound has 60 to 90 carbon atoms; R¹ represents a saturated or unsaturated aliphatic hydrocarbon group; and R² represents a saturated or unsaturated aliphatic hydrocarbon group optionally containing a ring structure or a substituent),
an ester thereof, or a salt thereof.

5. The method of producing macrophages according to claim 1 or 2, wherein
a cell culture vessel for culturing the hematopoietic progenitor cells has a surface coated with the TREAD signal activator.

6. The method of producing macrophages according to claim 1 or 2,
wherein
the hematopoietic progenitor cells are myeloid progenitor cells.

7. The method of producing macrophages according to claim 1 or 2, wherein
the hematopoietic progenitor cells are macrophage and dendritic cell progenitors, or common monocyte progenitors.

8. An agent for differentiation induction from hematopoietic progenitor cells into macrophages, the agent comprising a TREM2 signal activator.

9. A kit for differentiation induction from hematopoietic progenitor cells into macrophages, the kit comprising a cell culture vessel having a surface coated with a TREAD signal activator.

10. A method of culturing macrophages, the method comprising:
a culture step of culturing hematopoietic progenitor cells in presence of a TREAD signal activator.

11. An agent for promoting proliferation of macrophages, the agent comprising a TREAD signal activator.

12. A kit for promoting proliferation of macrophages, the kit comprising a cell culture vessel having a surface coated with a TREM2 signal activator.

13. A method of proliferating macrophages, the method comprising:
a culture step of culturing macrophages in presence of a TREM2 signal activator.

14. A macrophage having proliferative capacity after the 10th of subculture in a medium containing neither a macrophage colony-stimulating factor nor a granulocyte-macrophage colony-stimulating factor, but containing a TREM2 signal activator.

15. A macrophage whose differentiation is to be induced dependently on TREAD by culturing hematopoietic progenitor cells in a medium containing neither a macrophage colony-stimulating factor nor a granulocyte-macrophage-stimulating factor, but containing a TREAD signal activator.

16. A macrophage capable of proliferation after cryopreservation, in a medium containing neither a macrophage colony-stimulating factor nor a granulocyte-macrophage colony-stimulating factor, but containing a TREAD signal activator.

17. A macrophage whose culture supernatant after 24 hours of culture in a medium supplemented with lipopolysaccharide contains TNF-α, IL-6, and nitric oxide at concentrations of not more than 30% as compared to bone marrow derived macrophages induced from bone marrow cells with macrophage colony-stimulating factor.

18. A macrophage wherein expression of one or more genes selected from genes listed in Table 1 below is higher than in at least one selected from an alveolar macrophage, a BMDM, a BMDM induced to M1, a BMDM induced to M2, a Kupffer cell, a microglial cell, an osteoclast, and a peritoneal exudate macrophage.

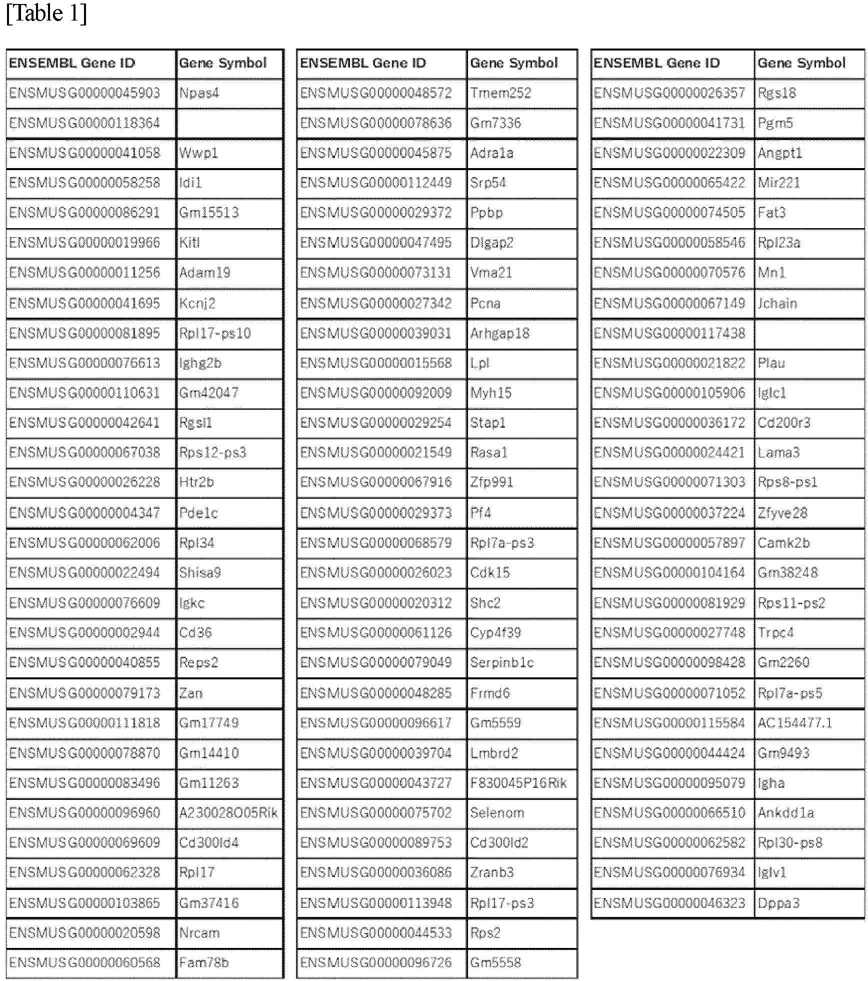

19. A macrophage wherein expression of one or more genes selected from genes listed in Table 2 below is lower than in at least one selected from an alveolar macrophage, a BMDM, a BMDM induced to M1, a BMDM induced to M2, a Kupffer cell, a microglial cell, an osteoclast, and a peritoneal exudate macrophage.
